Europäisches Patentamt

European Patent Office

Office européen des brevets

(11) Publication number: **0 461 297 A1**

# EUROPEAN PATENT APPLICATION

(21) Application number: 90112560.9

(22) Date of filing: 02.07.90

(51) Int. Cl.5: **C07C 21/18**, C07C 17/00

(30) Priority: 08.06.90 US 535273

(43) Date of publication of application:
**18.12.91 Bulletin 91/51**

(84) Designated Contracting States:
**BE DE FR GB IT NL**

(71) Applicant: **ATOCHEM NORTH AMERICA, INC.**
**Three Parkway**
**Philadelphia, Pennsylvania 19102(US)**

(72) Inventor: **Bolmer, Michael Sheppard**
**5033 Cold Springs Drive**
**Collegeville, Pennsylvania 19426(US)**
Inventor: **Elsheikh, Maher Yousef**
**784 North Wayne Avenue**
**Wayne, Pennsylvania 19087(US)**

(74) Representative: **Kraus, Walter, Dr. et al**
**Patentanwälte Kraus, Weisert & Partner**
**Thomas-Wimmer-Ring 15**
**W-8000 München 22(DE)**

(54) Production of vinylidene fluoride from 1,1-difluoroethane.

(57) 1,1-Difluoroethane is converted to vinylidene fluoride by contact with a $Cr_2O_3/Al_2O_3$ catalyst, in the presence of a co-reactant gas, at a reaction temperature of from about 300°C to about 700°C. The co-reactant gas advantageously comprises air, oxygen, carbon dioxide, or mixtures thereof.

EP 0 461 297 A1

## Field of the Invention

The invention relates to the dehydrogenation of fluorinated alkanes, and in particular to the dehydrogenation of 1,1-difluoroethane.

## Background of the Invention

Vinylidene fluoride is typically produced by dehydrochlorination of 1-chloro-1,1-difluoroethane. However, 1-chloro-1,1-difluoroethane is a relatively costly intermediate. 1,1-Difluoroethane, on the other hand, is relatively easily provided from the reaction of vinylidene chloride and hydrogen fluoride, such as described in U.S. Patent 3,978,145.

Vinylidene fluoride has been produced directly from 1,1-difluoroethane by dehydrogenation utilizing ultraviolet irradiation. Carmichael et al., J. Phys. Chem. 78(22), 2183-6 (1974). Conversion of 1,1-difluoroethane was very low, only 100 parts per million.

U.S. Patents 2,722,558 and 2,723,296 disclose a multi-step process for production of vinylidene fluoride from 1,1-difluoroethane. Chlorine gas is utilized as coreactant to convert 1,1-difluoroethane to 1-chloro-1,1-difluoroethane. The latter is thereafter dehydrochlorinated into vinylidene fluoride.

What is needed is a single step, direct method for converting 1,1-difluoroethane to vinylidene fluoride.

## Summary of the Invention

A process for production of vinylidene fluoride is provided, comprising flowing 1,1-difluoroethane over a cr2O3/Al2O3 catalyst at a reaction temperature from about 300° C to about 700° C with a co-reactant gas containing oxygen, carbon dioxide or a mixture thereof to obtain a product mixture comprising vinyl fluoride and vinylidene fluoride. Vinylidene fluoride is then recovered from the product mixture.

## Detailed Description of the Invention

We have found that 1,1-difluoroethane may be effectively converted to vinylidene fluoride by a gas phase dehydrogenation reaction in the presence of a solid $Cr_2O_3/Al_2O_3$ catalyst in the presence of a small amount of co-reactant gas containing oxygen, carbon dioxide or mixture thereof. Because of the substantial difference in the boiling points of the desired product, vinylidene fluoride (-86° C) and the starting material, 1,1-difluoroethane (-25° C), the two compounds may be easily separated by distillation. Unreacted 1,1-difluoroethane in the product may be recycled back to the reactor.

By "$Cr_2O_3/Al_2O_3$" is meant a catalyst compris-ing $Cr_2O_3$ supported on alumina. The chromium oxide may be incorporated into the alumina in any convenient manner. Preferably, the alumina is impregnated with a solution of chromium oxide.

1,1-Difluoroethane, in combination with the co-reactant gas is feed through a suitable reactor and over a catalyst bed containing the catalyst. The co-reactant gas, which is believed to promote catalyst longevity by continuously removing carbonaceous material from the catalyst surface, most advantageously comprises oxygen, carbon dioxide, or mixtures thereof. The co-reactant gas may also comprise a mixture of oxygen and/or carbon dioxide, optionally in admixture with one or more other gases which do not interfere with the dehydrogenation reaction. For example, the co-reactant gas may advantageously comprise atmospheric air, which principally contains nitrogen, oxygen and carbon dioxide.

The co-reactant gas is advantageously utilized in a mole ratio with 1,1-difluoroethane from about 0.04:1 to about 10:1, preferably from about 0.5:1 to about 4:1.

The reaction temperature in the vessel ranges from about 300° C to about 700° C, preferably from about 400° C to about 550° C.

The combined flow rates of 1,1-difluoroethane and co-reactant gas over the catalyst bed are advantageously selected to provide a total contact of all gases with the catalyst, of from about 1 second to about 60 seconds, preferably from about 5 second to about 20 seconds. While contact times longer than 60 seconds may be employed, they are not necessary. By "contact time" in this context is meant the catalyst bed volume, divided by the sum of the flow rates of 1,1-difluoroethane and the co-reactant gas contacted with the catalyst bed.

Vinylidene fluoride may be advantageously recovered from the reaction product by separating it from the major product, vinyl fluoride, by distillation. Vinylidene fluoride and vinyl fluoride have normal boiling points of -86° C and -72° C, respectively.

Separation may be advantageously accomplished by scrubbing hydrogen fluoride and $CO_2$ from the reaction product, removing air and water therefrom, and then finally feeding the reaction product to a distillation column. Vinylidene fluoride may be taken off as the distillation overhead product at a distillation column top temperature of, e.g., -3° C. Vinyl fluoride is removed as the column bottom product, at a column bottom temperature of, e.g., 14° C. The column is advantageously equipped with a condenser, cooled with, for instance, circulating refrigerated brine. The distillation column may be operated at a temperature below 300 psi, provided the condenser is adequately refrigerated to a lower temperature. The optimum balance between compressor cost and refrigeration cost may be readily ascertained by those skilled in the art by routine experimentation. Unreacted 1,1-difluoroethane may be distilled from the vinyl fluoride product and recycled back to the reactor.

The process of the invention is illustrated in the following non-limiting examples, wherein percentages of reactant feed gases are by volume, and percentages of 1,1-difluoroethane conversion to product are by mole. Comparative Example 1 demonstrates dehydrogenation of 1,1-difluoroethane in the absence of a catalyst.

## Comparative Example 1

Air and 1,1-difluoroethane in a molar ratio of 1:5 were fed from the top of a 1 inch outside diameter by 13 inch Hastelloy C empty tubular reactor at 500°C from valves located at the reactor top. The reaction products were withdrawn from the bottom of the reactor. Hydrogen fluoride was removed by passing the products up through a scrubbing tower containing a continuously circulating countercurrent alkaline stream, e.g., 1-5 N aqueous KOH. Other aqueous hydroxides, such as NaOH or $Ca(OH)_2$ may be utilized. The scrubbed organic products were then passed through a drying tower packed with a drying agent, such anhydrous calcium sulfate. The conversion of reactant to product was periodically checked by diverting the product from the drying tower to a gas chromatograph equipped with an electronic integrator. The mass balance was evaluated by passing the outlet gas from the gas chromatograph through a wet test meter. Gas chromatograph analysis indicated 15% conversion of 1,1-difluoroethane, with 1.6% selectivity for vinylidene fluoride and 98.4% selectivity for vinyl fluoride.

## Example 1

A catalyst composed of 19% $Cr_2O_3/Al_2O_3$ (32 grams, Harshaw) was placed in the same reactor utilized in Comparative Example 1. The catalyst was heated to 500°C with flowing nitrogen which was fed for 2 hours at the rate of 10 ml/min. 1,1-Difluoroethane and air in a mole ratio of 1:2.4 were then passed through the catalyst for a contact time of 26 seconds. The result was 75% conversion of 1,1-difluoroethane, with the following product selectivities: 0.7% vinylidene fluoride, 96.2% vinyl fluoride, and 3.2% unidentified heavier product.

## Example 2

1,1-Difluoroethane and $CO_2$ in a mole ratio of 1:3.7 were passed through the catalyst of Example 1 at 300°C for a contact time of 19 seconds. The result was 95% conversion of 1,1-difluoroethane, with 0.99% selectivity for vinylidene fluoride and 99.1% selectivity for vinyl fluoride.

## Example 3

1,1-Difluoroethane, $O_2$ and $CO_2$, in a mole ratio of 1:0.50:2.0, were passed through the catalyst of Example 1 at 400°C for a contact time of 11 seconds. The result was 98% conversion of 1,1-difluoroethane, with the following product selectivities: 5.0% vinylidene fluoride, 94.3% vinyl fluoride, and 0.7% unidentified heavier product.

## Example 4

1,1-Difluoroethane, $O_2$ and $CO_2$, in a mole ratio of 1:0.50:2.0, were passed through the catalyst of Example 1 at 500°C for a contact time of 10 seconds. A 98% conversion of 1,1-difluoroethane resulted, with the following product selectivities: 14.2% vinylidene fluoride and 85.8% vinyl fluoride.

The present invention may be embodied in other specific forms without departing from the spirit or essential attributes thereof and, accordingly, reference should be made to the appended claims, rather than to the foregoing specification, as indicating the scope of the invention.

## Claims

1. A process for producing vinylidene fluoride comprising flowing 1,1-difluoroethane over a $Cr_2O_3/Al_2O_3$ catalyst at a reaction temperature of from about 300°C to about 700°C with a co-reactant gas containing oxygen, carbon dioxide or mixture thereof to obtain a product mixture comprising vinyl fluoride and vinylidene fluoride, and recovering vinylidene fluoride from said product mixture.

2. A process according to claim 1 wherein the reaction temperature is from about 400°C to about 550°C.

3. A process according to claim 1 wherein the co-reactant comprises oxygen.

4. A process according to claim 1 wherein the co-reactant gas comprises carbon dioxide.

5. A process according to claim 1 wherein the co-reactant gas comprises air.

6. A process according to claim 1 wherein the molar ratio of 1,1-difluoroethane to co-reactant gas is from about 0.04:1 to about 10:1.

7. A process according to claim 6 wherein the molar ratio of 1,1-difluoroethane to co-reactant gas is from about 0.5:1 to about 4:1.

8. A process according to claim 1 wherein the contact time is from about 1 second to about 60 seconds.

9. A process according to claim 8 wherein the contact time is from about 5 seconds to about 20 seconds.

10. A process for producing vinylidene fluoride from 1,1-difluoroethane comprising continuously flowing gaseous 1,1-difluoroethane and a co-reactant gas in a molar ratio of from about 0.04:1 to about 10:1 over a $Cr_2O_3/Al_2O_3$ catalyst for a contact time of from about 1 second to about 60 seconds, at a reaction temperature of from 300°C to about 700°C to obtain a product mixture comprising vinyl fluoride and vinylidene fluoride, and recovering vinylidene fluoride from said product mixture.

11. A process according to claim 10 wherein the reaction temperature is from about 400°C to about 550°C.

12. A process according to claim 11 wherein the molar ratio of 1,1-difluoroethane to co-reactant gas is from about 0.5:1 to about 10:1.

13. A process according to claim 10 wherein the co-reactant gas comprises oxygen.

14. A process according to claim 10 wherein the co-reactant gas comprises carbon dioxide.

15. A process according to claim 10 wherein the co-reactant gas comprises air.

16. A process according to claim 11 wherein the contact time is from about 5 seconds to about 20 seconds.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| E | EP-A-0 406 748 (ATOCHEM NORTH AMER-ICA)September 1991<br>* claims 36-45 * * | 1-16 | C 07 C 21/18<br>C 07 C 17/00 |
| A,D | US-A-2 723 296 (I. LITANT ET AL)<br>* claim 3 * * | | |

TECHNICAL FIELDS SEARCHED (Int. Cl.5)

C 07 C

The present search report has been drawn up for all claims

| Place of search | Date of completion of search | Examiner |
|---|---|---|
| The Hague | 21 October 91 | VAN GEYT J.J.A. |